Europäisches Patentamt

European Patent Office

Office européen des brevets

(11) Veröffentlichungsnummer: **0 113 420**
**A1**

(12) ## EUROPÄISCHE PATENTANMELDUNG

(21) Anmeldenummer: **83111871.6**

(22) Anmeldetag: **26.11.83**

(51) Int. Cl.³: **A 61 F 5/00**

(30) Priorität: 09.12.82 DE 8234527 U
09.12.82 DE 8234528 U

(43) Veröffentlichungstag der Anmeldung:
18.07.84 Patentblatt 84/29

(84) Benannte Vertragsstaaten:
AT BE CH DE FR GB IT LI NL SE

(71) Anmelder: Roehr, Oskar W.K.
Windmühlenstieg 15
D-2000 Hamburg 52(DE)

(72) Erfinder: Roehr, Oskar W.K.
Windmühlenstieg 15
D-2000 Hamburg 52(DE)

(74) Vertreter: Patentanwälte Dipl.-Ing. J. Richter Dipl.-Ing. F.
Werdermann
Neuer Wall 10
D-2000 Hamburg 36(DE)

(54) Einrichtung zum Erwärmen oder Kühlen von liegenden oder sitzenden Personen.

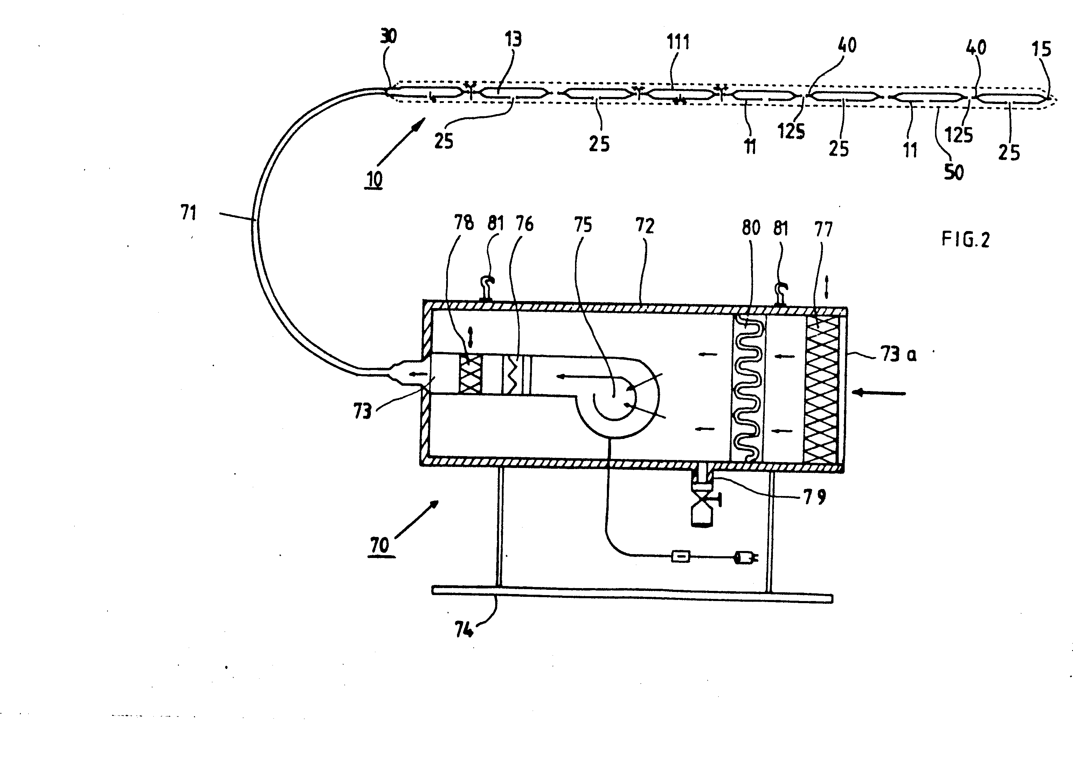

(57) Die Erfindung betrifft eine Bedeckung für Personen, um diesen wahlweise Warmluft oder Kaltluft zuführen zu können, wobei für einen gleichmäßigen Luftaustritt die an eine Warmluft und/oder Kaltluft erzeugende Einrichtung (70) angeschlossene Decke (10) mit einer Anzahl von Luftaustrittsöffnungen (25) versehen ist, während im Innenraum der aus zwei randverschweißten Lagen (11,111) bestehenden Decke (10) mittels Schweiß- oder Klebabschnitten eine Vielzahl von miteinander verbundenen luftkanalartigen Abschnitten (21) zur Luftführung zu den Luftaustrittsöffnungen (25) ausgebildet ist (Fig. 2).

./...

FIG.2

## Einrichtung zum Erwärmen oder Kühlen von liegenden oder sitzenden Personen

Die Erfindung betrifft eine Einrichtung zum Erwärmen oder Kühlen von liegenden oder sitzenden Personen.

Zum Schutz gegen eine Abkühlung des menschlichen Körpers ist die Verwendung elektrisch beheizbarer Bedeckungen in Form von Schlaf- oder Bettdecken bekannt, die jedoch den Nachteil besitzen, daß eine trockene Wärme erzeugt wird, die oftmals zu einer erhöhten Schweißabsonderung führen kann und sich z.B. bei in einem Krankenhaus- oder Klinik-Bett liegenden Patienten insofern als nachteilig auswirkt, als trotz der Wärmezufuhr die Wärme als unangenehm empfunden wird , wobei auch noch das Sicherheitsproblem derartig elektrisch beheizbarer Decken hinzu kommt, auch wenn diese Decken mit Überhitzungsthermostaten und -schaltern ausgerüstet sind, die bei Erreichen einer bestimmten Temperatur die weitere Stromzufuhr unterbrechen sollen. Hinzu kommt noch, daß derartige beheizbare, wärmeabgebende Decken technisch nicht so ausgebildet sind, daß auch Kühlluft abgegeben werden kann, wenn dies erforderlich sein sollte.

Die Erfindung löst die Aufgabe, eine Einrichtung zu schaffen, die eine Bedeckung in Form einer Decke für liegende oder sitzende Personen und eine Warmluft- und/oder Kaltlufterzeugungseinrichtung umfaßt, vermittels der keine trockene Warmluft, sondern erwärmte Normalluft der Decke und somit der Person zugeführt wird und die auch geeignet ist, Kaltluft abzugeben, wenn eine Kühlung der betreffenden Person erforderlich ist, so daß die Bedeckung bei entsprechender Ausgestaltung auch zu Heilzwecken herangezogen werden kann. Des weiteren soll erreicht werden, daß eine gleichmäßige Beaufschlagung der Person durch Luft erfolgt und die Ausbildung eines Überdruckes im Zwischenraum zwischen Decke und dem Körper der Person vermieden wird.

Zur Lösung der Aufgabe wird erfindungsgemäß eine Einrichtung zum Erwärmen oder Kühlen von liegenden oder sitzenden Personen vorgeschlagen, die aus

a) einer doppelwandigen Decke aus zwei im Randbereich durch Schweiß- oder Klebverbindungen unter Ausbildung eines geschlossenen Innenraumes zusammengehaltenen Lagen aus schweißbarer Kunststoffolie oder einem mit einer Kunststoffolie kaschierten Gewebe, die unter Ausbildung eines Warm- oder Kaltluft führenden Kanalsystems aus einer Vielzahl von miteinander verbundenen luftkanalartigen Abschnitten in dem Deckeninnenraum abschnittsweise bzw. punktartig über Schweiß- oder Klebabschnitte miteinander verbunden sind, wobei eine oder beide Lagen mit Luftaustrittsöffnungen und der Deckeninnenraum mit einem im Randbereich der Decke oder an einer der beiden Lagen befestigten Luftzuführungsstutzen versehen ist,

b) einer Anzahl von Luftdurchtrittsdurchbrechungen in den Schweiß- und Klebeabschnitten und

c) einer mit dem Innenraum der Decke über einen mit dem Luftzuführungsstutzen verbundenen Luftzuführungsschlauch verbundenen Warm- oder Kaltlufterzeugungs- und Zuführungsvorrichtung aus einem mit einem Standgestell und/oder einer Aufhängung versehenen Gehäuse, in dessen Innenraum ein Saug-Druck-Gebläse, eine im Bereich der Luftaustrittsöffnung des Gehäuses liegende Heizeinrichtung und mindestens ein Reinigungs- und/oder Luftbefeuchtungsfilter und/oder ein Sauerstoff- bzw. Mischgaszuführungsstutzen und/oder ein Kühlaggregat im Bereich der Luftansaugöffnung angeordnet sind,

besteht.

Des weiteren sieht die Erfindung eine Einrichtung zum Erwärmen oder Kühlen von liegenden oder sitzenden Personen vor, die aus

a) einer doppelwandigen Decke aus zwei im Randbereich durch Schweiß- oder Klebverbindungen unter Ausbildung eines geschlossenen Innenraumes zusammengehaltenen Lagen aus schweißbarer Kunststoffolie oder einem mit einer Kunststoffolie kaschierten Gewebe, die unter Ausbildung eines Warm- oder Kaltluft führenden Kanalsystems aus einer Vielzahl von miteinander verbundenen luftkanalartigen Abschnitten in dem Deckeninnenraum abschnittsweise bzw. punktartig über Schweiß- oder Klebabschnitte miteinander verbunden sind, wobei eine oder beide Lagen mit Luftaustrittsöffnungen und der Deckeninnenraum mit einem im Randbereich der Decke oder an einer der beiden Lagen befestigten Luftzuführungsstutzen versehen ist,

b) einer Anzahl von Luftdurchtrittsdurchbrechungen in den Schweiß- und Klebeabschnitten und

c) einer die doppelwandige Decke allseitig umgebenden Hülle aus einem luftdurchlässigen und luftverteilenden Gewebe und

d) einer mit dem Innenraum der Decke über einen mit dem Luftzuführungsstutzen verbundenen Luftzuführungsschlauch verbundenen Warm- oder Kaltlufterzeugungs- und Zuführungsvorrichtung aus einem mit einem Standgestell und/oder einer Aufhängung versehenen Gehäuse, in dessen Innenraum ein Saug-Druck-Gebläse, eine im Bereich der Luftaustrittsöffnung des Gehäuses liegende Heizeinrichtung und mindestens ein Reinigungs- und/oder Luftbefeuchtungsfilter und/oder ein Sauerstoff- bzw. Mischgaszuführungsstutzen und/oder ein Kühlaggregat im Bereich der Luftansaugöffnung angeordnet sind,

besteht.

Mit einer derart ausgebildeten Einrichtung ist es möglich, der die Decke der Einrichtung benutzenden Person wahlweise Warmluft oder Kaltluft vermittels des mit der Decke verbundenen Gebläses mit einer Heizeinrichtung zuzuführen. Im Körperbereich wird dann keine trockene Warmluft erzeugt, wie bei den bekannten, elektrisch beheizbaren Decken, sondern eine Warmluft, die ein gewisses Maß an Luftfeuchtigkeit aufweist, deren Höhe sich jeweils nach der Luftfeuchtigkeit der Umgebungsluft richtet, von der die Luft zur Warmlufterzeugung entnommen wird, wobei auch eine zusätzliche Befeuchtung der zugeführten Luft vorgenommen werden kann. Bei Nichtinbetriebnahme der Heizeinrichtung der Warmlufterzeugungsvorrichtung wird über das Gebläse Kaltluft der Decke zugeführt. Ist die Warmlufterzeugungsvorrichtung mit mehreren
Anschlußstutzen versehen, so können mit einer einzigen Warmlufterzeugungsvorrichtung mehrere Decken mit Warmluft oder
bei abgeschalteter Heizeinrichtung mit Kaltluft versorgt
werden. In Verbindung mit einem die Decke umhüllenden Bezug
aus einem geeigneten Gewebe ist ein einwandfreier Luftdurchtritt durch das Gewebe bei gleichzeitiger gleichmäßiger
Luftverteilung gewährleistet. Die aus der Decke austretende
Luft verteilt sich gleichmäßig zu beiden Seiten der Decke
in den Zwischenräumen  zwischen der Decke und der Hülle bzw.
dem Bezug.

Der Anwendungsbereich einer derart ausgebildeten Einrichtung
erstreckt sich auf die verschiedensten Möglichkeiten. Neben
der Verwendung der Wärmedecke in Haushalten findet die
Einrichtung auch Verwendung in Krankenhäusern und Kliniken, in denen Patienten oftmals einer besonderen Wärmezufuhr
bedürfen, da die  Krankenzimmer nicht übermäßig erwärmt
werden und insbesondere ältere Patienten ein besonders
ausgeprägtes Wärmeverlangen haben.

Darüber hinaus ist es auch möglich, im Bedarfsfalle den
Patienten Kaltluft zuzuführen, was besonders vorteilhaft
an sehr warmen Tagen ist und eine Kühlung oftmals zu einer
Verbesserung des allgemeinen Wohlbefindens und darüber
hinaus auch in bestimmten Fällen, wie bei Verbrennungen,
zu einer Linderung beiträgt. Wird die Decke bei entsprechender Formgebung, z.B. als Hülle oder als kleines Kissen ,
verwendet und wird dann der Decke gekühlte Luft zugeführt,
so kann die Decke zur Unterstützung eines Heilungsprozesses
dienen und zur lokalen Behandlung am Körper herangezogen
werden, z.B. in der Ausgestaltung als Hülle um die Füße
eines Patienten gelegt als Ersatz für einen Wadenwickel oder als kleines Kissen auf die Brust in der Herzgegend gelegt zur Linderung von Herzbeschwerden.

Des weiteren läßt sich die Einrichtung in warmen Gegenden mit tropisch heißem Klima zur Kühlung von Personen anwenden und eine kostspielige Klimaanlage mit hohen Betriebskosten dadurch ersetzen. Bei unterkühlten Personen findet die Einrichtung mit Warmluftzufuhr Anwendung.

Vorteilhafte Weiterbildungen der Erfindung sind in den Unteransprüchen beschrieben.

Im folgenden wird der Gegenstand der Erfindung in den Zeichnungen erläutert. Es zeigt

Fig. 1  in einer Ansicht von oben eine aus einer Decke und einer Warmlufterzeugungsvorrichtung bestehende Einrichtung,

Fig. 2  einen senkrechten Schnitt gemäß Linie II-II in Fig. 1 und

Fig. 3  in einer schaubildlichen Seitenansicht eine zu einem Schlafsack oder Hülle umgewandelte Decke.

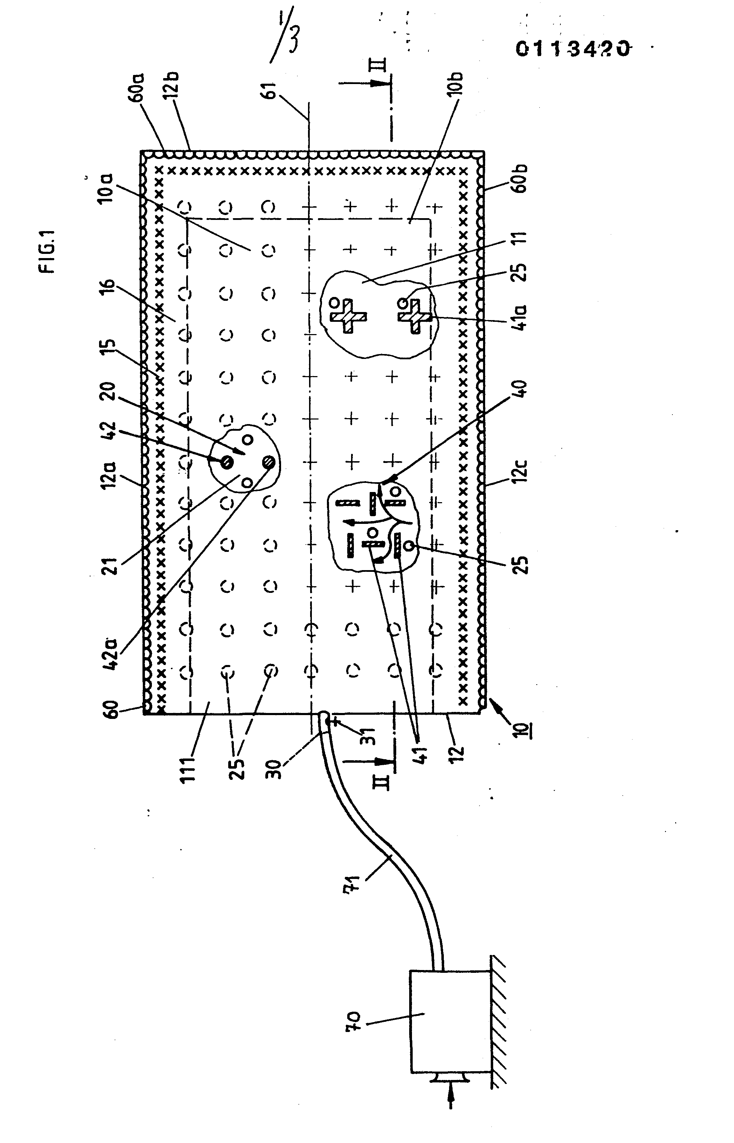

Die in Fig. 1 und 2 dargestellte Einrichtung besteht aus einer Decke 10 und einer Warmlufterzeugungsvorrichtung 70.

Die Decke 10 ist doppelwandig ausgebildet und besteht aus zwei Lagen 11,111 aus vorzugsweise rechteckförmigen Zuschnitten aus schweißbarer Kunststoffolie oder einem mittels einer Kunststoffolie kaschierten Gewebe. Die beiden Lagen 11,111 sind im Bereich ihrer umlaufenden Ränder 12 miteinander verschweißt oder verklebt. Die Randschweiß- oder Klebverbindung ist bei 15 angedeutet (Fig.1).

Die Decke 10 kann auch aus einem einzigen, rechteckförmigen Zuschnitt bestehen, der mittig und quer zu seiner Längsrichtung gefaltet ist, so daß die beiden Zuschnittshälften aufeinander zu liegen kommen und so die beiden Lagen 11,111 bilden, die dann in einem Deckenendbereich über die Faltlinie miteinander verbunden sind, während die drei verbleibenden Ränder 12a,12b,12c miteinander verschweißt oder verklebt sind.

Die beiden miteinander randverschweißten oder randverklebten Lagen 11,111 umschließen einen Innenraum 13 und sind unter Ausbildung eines Warm- oder Kaltluft führenden Kanalsystems 20 aus einer Vielzahl von miteinander verbundenen luftkanalartigen Abschnitten 21 in dem Innenraum 13 abschnittsweise, was in Fig. 1 bei 41 angedeutet ist, oder punktartig, wie bei 42 angedeutet, über Schweiß- oder Klebabschnitte 40 miteinander verbunden. Die punktartigen Schweißstellen können als scheibenartige Schweißabschnitte 42a ausgebildet sein; jedoch können auch andersartig ausgebildete Schweißabschnitte, die bei 41a angedeutet sind, zur Verbindung der beiden Lagen 11,111 und zur Ausbildung des Warm- oder Kaltluft führenden Kanalsystems 20 dienen. Diese Schweißabschnitte 40 sind zueinander versetzt angeordnet, so daß die durch den Innenraum 13 der Decke 10 geführte Luft durch den gesamten Deckeninnenraum 13 hindurchströmen kann. Auch hier ist vorteilhafterweise eine versetzte Anordnung und Zuordnung der Verbindungsstege vorgesehen.

Die Zufuhr von Warm- oder Kaltluft in den Innenraum 13 der Decke 10 erfolgt über einen Luftzuführungsstutzen 30, der über eine flexible Schlauchleitung 71 mit der Warmlufterzeugungsvorrichtung 70 verbunden ist.

Um die in den Innenraum 13 der Decke 10 zugeführte Warm- oder Kaltluft der mit der Decke 10 bedeckten Person zuführen zu können, weist die Lage 11 oder beide Lagen 11,111 im Bereich der luftkanalartigen Abschnitte 21 Luftaustrittsöffnungen 25 auf, die über die gesamte Fläche der Lage 11 und/oder der beiden Lagen 11,111 verteilt sind. Um den Luftaustritt durch die Luftaustrittsöffnungen 25 in keiner Weise zu schmälern, sind die Luftaustrittsöffnungen 25 benachbart bzw. im Bereich der Schweiß- oder Klebabschnitte 40 vorgesehen (Fig. 1).

Wie Fig. 2 zeigt, weisen die Schweißabschnitte 40 Durchbrechungen 125 auf, durch die die aus den Öffnungen 25 austretende Luft gleichmäßig über die Oberfläche der Decke 10 verteilt wird. Dies ist besonders vorteilhaft, wenn die Decke 10 nur in ihrer Lage 11 Luftaustrittsöffnungen 25 aufweist und von einer Hülle 50 umgeben ist. Die aus den Öffnungen 25 austretende Luft strömt durch die Durchbrechungen 125 auf die Oberseite der Decke 10 und verteilt sich hier gleichmäßig zwischen der Decke 10 und dem oberen Hüllenabschnitt, so daß ein guter Erwärmungs- oder Kühleffekt erreicht wird. Außerdem tragen die Durchbrechungen 125 zur Vermeidung eines Unterdruckes zwischen der Decke 10 und dem Körper der die Decke benutzenden Person bei.

In dem Warm- oder Kaltluftzuführungsstutzen 30 bzw. in dem Verbindungsschlauch 70 kann zusätzlich ein Sicherheitsventil 31 vorgesehen sein, um zu vermeiden, daß sich im Innenraum 13 der Decke 10 ein Überdruck bilden kann.

Der Warm- oder Kaltluftstutzen 30 ist an einer der beiden Lagen 11 oder 111 oder im Randbereich 12 der Decke 10 angeschlossen, so wie dies in Fig. 1 und 2 dargestellt ist.

Um eine gleichmäßige Luftmengenverteilung im Deckeninnenraum 13 zu erreichen, ist benachbart zum umlaufenden Rand 12 an der die Luftaustrittsöffnungen 25 aufweisenden Lage 11 und/oder den Lagen 11,111 ein luftaustrittsöffnungsfreier Abschnitt 16 ausgebildet, durch den die einströmende Luft ungehindert hindurchströmen kann, um von dort aus dann in den Bereich der Luftaustrittsöffnungen 25 zu gelangen, durch die dann die Luft entweichen kann. Vorzugsweise sind die beiden Lagen 11,111 im Bereich des luftaustrittsöffnungsfreien Abschnittes 16 nicht über Schweiß- oder Klebabschnitte 40 miteinander verbunden, so daß die Luft ungehindert durch diesen Abschnitt 16 hindurchströmen kann.

Die Decke 10 findet auch als Einziehdecke in einem Überzug Verwendung bzw. ist mit einer Hülle 50 versehen (Fig. 2).

Diese Hülle 50 besteht aus einem luftdurchlässigen Gewebe, damit die aus den Luftaustrittsöffnungen 25 ausströmende Luft an den Körper der die Decke benutzenden Person gelangen kann. Hierzu sind auch handelsübliche Decken oder Kissenbezüge geeignet.

Um die Decke 10 auch als Schlafsack oder Umhüllung benutzen zu können, ist die Decke 10 mit einem oder mehreren Reißverschlüssen, Klettenverschlüssen od.dgl. 60,60a,60b versehen, wobei sich die Reißverschlüsse über zwei oder drei aneinandergrenzende Randbereiche der Decke 10 erstrecken, so daß eine Einschlupföffnung bzw. zwei Öffnungen verbleiben.

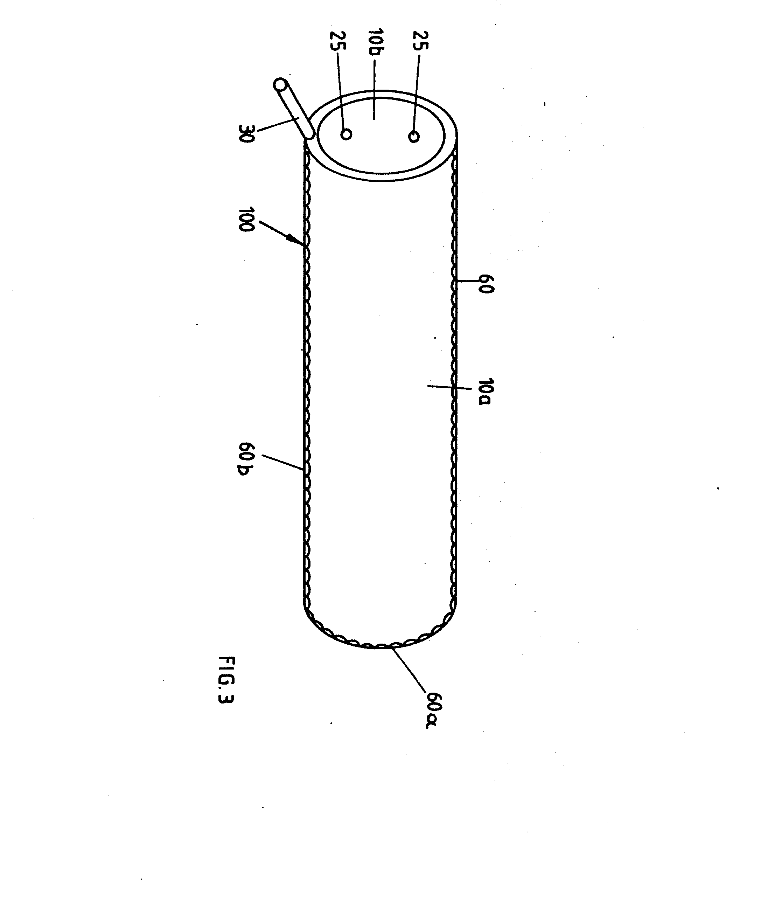

Um aus der Decke 10 einen Schlafsack 100 entsprechend Fig. 3 zu bilden, wird die Decke 10 um ihre Längsmittellinie 61 so gefaltet, daß die beiden Deckenhälften 10a,10b aufeinander zu liegen kommen. Hierauf wird dann die offene Längsseite und der bodenseitige Bereich mittels der Reißverschlüsse oder anderen geeigneten Verbindungsmitteln geschlossen. Auf diese Weise können auch Hüllen geschaffen werden, um Gliedmaße, wie Arme und Beine, zu umschließen.

Die Warmlufterzeugungsvorrichtung 70 besteht nach Fig. 2 aus einem mit einem Standgestell 74 oder einer Aufhängung 81 versehenen Gehäuse 72 mit einer Luftansaugöffnung 72a und einem Luftaustrittsstutzen 73, an den der Verbindungsschlauch 71 der Decke 10 angeschlossen ist. In dem Innenraum des Gehäuses 72 ist ein Gebläse 75 derart angeordnet, daß von diesem durch die Gehäuseöffnung 72a Luft angesogen und über den Verbindungsschlauch 71 in den Innenraum 13 der Decke 10 gedrückt wird.

Im Innenraum des Gehäuses 72 der Warmlufterzeugungseinrichtung 70 ist ferner im Bereich der Luftaustrittsöffnung 72a eine Heizvorrichtung 76 in Form einer Heizspirale od.dgl. angeordnet, vermittels der die angesogene Luft erwärmt bzw. aufgeheizt wird. Der Heizvorrichtung 76 ist ein Luft-befeuchtungsfilter 78 nachgeschaltet, über den die durchströmende Luft befeuchtet wird, falls dies erforderlich sein sollte. Ein oder mehrere weitere Filter 77 können als Luftreinigungsfilter noch in dem Gehäuse 72 untergebracht sein. Alle Filter 77,78 sind als auswechselbare Einschubkassetten ausgebildet, um das Auswechseln der Filter zu erleichtern. An dem Gehäuse 72 ist ebenfalls ein Stutzen 79 angebracht, um der Gebläseluft Sauerstoff oder andere gasförmige Medien beizumischen.

Die Geschwindigkeit des Gebläses, Lüfters oder Ventilators 75 und auch die Heizvorrichtung 76 ist stufenlos regelbar. Ist die Heizvorrichtung 76 nicht eingeschaltet, so wird von dem Gebläse Kaltluft, d.h. Luft mit der Temperatur der Umgebungsluft, angesogen und der Decke 10 zugeführt. Mit einem eingebauten Kühlaggregat läßt sich die Temperatur noch herabsetzen.

Sowohl die Warmlufterzeugungseinrichtung als auch die Kaltlufterzeugungseinrichtung können in gesonderten Bauteilen untergebracht sein, die baukastenartig vermittels an sich bekannter Befestigungsmittel miteinander verbunden werden können, so daß mit einem Gerät wahlweise Warmluft und Kaltluft erzeugt werden kann. Die Unterbringung der Warmlufterzeugungseinrichtung und der Kaltlufterzeugungseinrichtung in getrennten Bauteilen erbringt den Vorteil, daß wahlweise das eine oder das andere Bauteil einzeln verwendet werden kann.

Patentansprüche

1. Einrichtung zum Erwärmen oder Kühlen von liegenden oder sitzenden Personen, dadurch gekennzeichnet, daß die Einrichtung aus

   a) einer doppelwandigen Decke (10) aus zwei im Randbereich (12; 12a,12b,12 c) durch Schweiß- oder Klebverbindungen (15) unter Ausbildung eines geschlossenen Innenraumes (13) zusammengehaltenen Lagen (11,111) aus schweißbarer Kunststoffolie oder einem mit einer Kunststoffolie kaschierten Gewebe, die unter Ausbildung eines Warm- oder Kaltluft führenden Kanalsystems (20) aus einer Vielzahl von miteinander verbundenen luftkanalartigen Abschnitten (21) in dem Deckeninnenraum (13) abschnittsweise (41) bzw. punktartig (42) über Schweiß- oder Klebabschnitte (40) miteinander verbunden sind, wobei eine (11) oder beide Lagen (11,111) mit Luftaustrittsöffnungen (25) und der Deckeninnenraum (13) mit einem im Randbereich der Decke (10) oder an einer der beiden Lagen (11,111) befestigten Luftzuführungsstutzen(30) versehen ist,

   b) einer Anzahl von Luftdurchtrittsdurchbrechungen (125) in den Schweiß- und Klebabschnitten (40) und

   c) einer mit dem Innenraum (13) der Decke (10) über einen mit dem Luftzuführungsstutzen (30) verbundenen Luftzuführungsschlauch (71) verbundenen Warm- oder Kaltlufterzeugungs- und Zuführungsvorrichtung (70) aus einem mit einem Standgestell (74) und/oder einer Aufhängung (81) versehenen Gehäuse (72), in dessen Innenraum ein Saug-Druck-Gebläse (75), eine im Bereich der Luftaustrittsöffnung (73) des Gehäuses (72) liegende Heizeinrichtung (76) und mindestens ein Reinigungs- und/oder Luftbefeuchtungsfilter (77,78) und/oder ein Sauerstoff- bzw. Mischgaszuführungsstutzen (79) und/oder ein Kühlaggregat (80) im Bereich der Luftansaugöffnung (73a) angeordnet sind,

besteht.

2. Einrichtung zum Erwärmen oder Kühlen von liegenden oder sitzenden Personen, dadurch gekennzeichnet, daß die Einrichtung aus

a) einer doppelwandigen Decke (10) aus zwei im Randbereich (12; 12a,12b,12 c) durch Schweiß- oder Klebverbindungen (15) unter Ausbildung eines geschlossenen Innenraumes (13) zusammengehaltenen Lagen (11,111) aus schweißbarer Kunststoffolie oder einem mit einer Kunststoffolie kaschierten Gewebe, die unter Ausbildung eines Warm- oder Kaltluft führenden Kanalsystems (20) aus einer Vielzahl von miteinander verbundenen luftkanalartigen Abschnitten (21) in dem Deckeninnenraum (13) abschnittsweise (41) bzw. punktartig (42) über Schweiß- oder Klebabschnitte (40) miteinander verbunden sind, wobei eine (11) oder beide Lagen (11,111) mit Luftaustrittsöffnungen (25) und der Deckeninnenraum (13) mit einem im Randbereich der Decke (10) oder an einer der beiden Lagen (11,111) befestigten Luftzuführungsstutzen(30) versehen ist,

b) einer Anzahl von Luftdurchtrittsdurchbrechungen (125) in den Schweiß- und Klebeabschnitten (40) und

c) einer die doppelwandige Decke (10) allseitig umgebenden Hülle (50) aus einem luftdurchlässigen und luftverteilenden Gewebe und

d) einer mit dem Innenraum (13) der Decke (10) über einen mit dem Luftzuführungsstutzen (30) verbundenen Luftzuführungsschlauch (71) verbundenen Warm- oder Kaltlufterzeugungs- und Zuführungsvorrichtung (70) aus einem mit einem Standgestell (74) und/oder einer Aufhängung (81) versehenen Gehäuse (72), in dessen Innenraum ein Saug-Druck-Gebläse (75), eine im Bereich der Luftaustrittsöffnung (73) des Gehäuses (72) liegende Heizeinrichtung (76) und mindestens ein Reinigungs- und/oder Luftbefeuchtungsfilter (77,78) und/oder ein Sauerstoff- bzw. Mischgaszuführungsstutzen (79) und/oder ein Kühlaggregat (80) im Bereich der Luftansaugöffnung (73a) angeordnet sind,

besteht.

3. Einrichtung nach Anspruch 1 und 2, dadurch gekennzeichnet, daß benachbart zum umlaufenden Rand (12) bzw. der Randverschweißung oder -verklebung (15) der beiden Lagen (11,111) der Decke (10) ein luftaustrittsöffnungsfreier Abschnitt (16) ausgebildet ist.

4. Einrichtung nach Anspruch 1 bis 3 dadurch gekenn- zeichnet, daß die Luftaustrittsöffnungen (25) in der Decke (10) beliebig verteilt, z.B. zu dem Deckenrandbereich (12;12a,12b,12c) zunehmend angeordnet sind.

5. Einrichtung nach Anspruch 1 bis 4 , dadurch gekennzeichnet, daß die Decke (10) großflächig oder in Kissenform ausgebildet ist.

6. Einrichtung nach Anspruch 1 bis 5 , dadurch gekennzeichnet, daß der Warm- oder Kaltluftzuführungsstutzen (30) mit einem Sicherheitsventil (31) versehen ist.

7. Einrichtung nach Anspruch 1 bis 6 , dadurch gekennzeichnet, daß zur Ausbildung der luftkanalartigen Abschnitte (21) in dem Innenraum (13) der Decke (10) die die beiden Lagen (11,111) miteinander verbindenden Schweiß- oder Kleb- abschnitte (40) steg- (41a) oder scheibenartig (42a) ausgebildet sind, in denen die Luftdurchtrittsdurchbrechungen (125) angeordnet sind.

8. Einrichtung nach Anspruch 1 bis 7 , dadurch gekennzeichnet, daß die Luftaustrittsöffnungen (25) in der Lage (11) oder Lagen (11,111) der Decke (10) benachbart bzw. im Bereich der Schweiß- oder Klebabschnitte (40) vorgesehen sind.

9. Einrichtung nach Anspruch 1 bis 8 , dadurch gekennzeichnet, daß die Decke (10) in einer Hülle (50) aus einem luftdurchlässigen und luftverteilenden Gewebe angeordnet ist.

10. Einrichtung nach Anspruch 1 bis 9, dadurch gekennzeichnet, daß die Decke (10) an mindestens drei aneinandergrenzenden Rändern mit Reißverschlüssen (60,60a,60b) oder anderen geeigneten Verbindungsmitteln versehen ist und nach der Faltung um ihre Mittellängsachse (61) und nach Aufeinanderklappen der beiden Deckenhälften (10a,10b) diese mittels der Reißverschlüsse oder mehreren geeigneten Verbindungsmitteln, wie Klettenverschlüsse od.dgl.(60,60a,60b) zusammengehalten sind.

11. Einrichtung nach Anspruch 1 bis 10, dadurch gekennzeichnet, daß der Reinigungsfilter (77) und der Luftbefeuchtungsfilter (78) als in das Gehäuse (72) einschiebbare Austauschkassetten ausgebildet sind.

12. Einrichtung nach Anspruch 1 bis 11, dadurch gekennzeichnet, daß der Luftbefeuchtungsfilter (78) der Heizeinrichtung (76) nachgeschaltet ist.

13. Einrichtung nach Anspruch 1 bis 12, dadurch gekennzeichnet, daß das Kühlaggregat (80) dem Gebläse (75) vorgeschaltet ist.

14. Einrichtung nach Anspruch 1 bis 13, dadurch gekennzeichnet, daß das Gehäuse (72) der Warmlufterzeugungseinrichtung (70) im Bereich des Luftaustrittsstutzens (73) mehrere Anschlußstutzen aufweist.

15. Decke für eine Einrichtung zum Erwärmen oder Kühlen von liegenden oder sitzenden Personen nach den Ansprüchen 1 bis 14, dadurch gekennzeichnet, daß die Decke (10) doppelwandig ausgebildet ist und aus zwei im Randbereich (12;12a,12b,12c) durch Schweiß- oder Klebverbindungen (15) unter Ausbildung eines geschlossenen Innenraumes (13) zusammengehaltenen Lagen (11,111) aus schweißbarer Kunststoffolie oder einem mit einer Kunststoffolie kaschierten Gewebe besteht, die unter Ausbildung eines Warm- oder Kaltluft führenden Kanalsystems (20) aus einer Vielzahl von miteinander verbundenen luftkanalartigen Abschnitten (21) in dem Deckeninnenraum (13) abschnittsweise (41) bzw. punktartig (42) über Schweiß- oder Klebabschnitte miteinander verbunden sind, wobei eine (11) oder beide Lagen (11,111) mit Luftaustrittsöffnungen (25) versehen und der Deckeninnenraum (13) mit einem im Randbereich der Decke (10) oder an einer der beiden Lagen (11,111) befestigten Warm- oder Kaltluftzuführungsstutzen (30) verbunden ist.

16. Einrichtung nach Anspruch 1 bis 15, dadurch gekennzeichnet, daß die Warmluft- und Kaltlufterzeugungseinrichtung (70) aus einem die Warmlufterzeugungseinrichtung aufnehmenden Bauteil und aus einem die Kaltlufterzeugungseinrichtung aufnehmenden Bauteil besteht und daß beide Bauteile baukastenartig mittels Verbindungseinrichtungen miteinander verbindbar sind.

FIG.1

FIG.2

30  13  111  40  40  15

10  25  25  11  125  25  11  125  25  50

71

78  81  76  75  72  80  81  77

73a

73

79

70

74

FIG.3

Europäisches
Patentamt

EUROPÄISCHER RECHERCHENBERICHT

0113420
Nummer der Anmeldung

EP 83 11 1871

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X,A | DE-A-2 226 444  (BORN)  * Ganzes Dokument * | 1,2,5, 7,8,9, 12,15 | A 61 F  5/00 |
| X,A | FR-A-1 327 862  (PHILIBERT)  * Ganzes Dokument * | 1,2,5, 7,9,15 | |
| X,A | US-A-2 512 559  (WILLIAMS) * Anspruch 1; Figuren 1, 7 * | 1,5,15 | |
| A | US-A-3 867 939  (MOORE et al.) * Spalte 6, Zeilen 26-29 * | 1 | |
| A | US-A-2 235 966  (SUMMERS) * Figuren 1, 3 * | 1,11 | RECHERCHIERTE SACHGEBIETE (Int. Cl. 3) |
| A | US-A-3 866 612  (BUKER)  * Figur 2 * | 1,13, 14 | A 61 F  5/00 |
| A | US-A-2 093 834  (GAUGLER) * Figur 6 * | 1,16 | |
| A | GB-A-1 035 893  (LIDDLE) | | |
| A | US-A-2 590 026  (MARX) | | |

--- -/-

Der vorliegende Recherchenbericht wurde für alle Patentansprüche erstellt.

| Recherchenort BERLIN | Abschlußdatum der Recherche 29-02-1984 | Prüfer KANAL P K |
|---|---|---|

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03.82

## 0113420

Nummer der Anmeldung

EP 83 11 1871

## EINSCHLÄGIGE DOKUMENTE

Seite 2

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A | CH-A- 149 244 (POPP) | | |

-----

RECHERCHIERTE SACHGEBIETE (Int. Cl. ³)

Der vorliegende Recherchenbericht wurde für alle Patentansprüche erstellt.

| Recherchenort | Abschlußdatum der Recherche | Prüfer |
|---|---|---|
| BERLIN | 29-02-1984 | KANAL P K |